# EUROPEAN PATENT SPECIFICATION

(11) **EP 2 364 744 B1**
(45) Date of publication and mention of the grant of the patent: **22.08.2012**
(21) Application number: 10155956.5
(22) Date of filing: 09.03.2010
(51) Int. Cl.: A61M 5/50, A61M 5/32

(54) **Safety needle cap**
Sicherheitsnadelkappe
Capuchon d'aiguille de sécurité

(43) Date of publication of application: 14.09.2011
(73) Proprietor: Lin, Ming-Tien, Nantou Hsien (TW); Teng, Hsi-Hsiung, Kaohsiung (TW)
(72) Inventor: Lin, Ming-Tien, Nantou Hsien (TW); Teng, Hsi-Hsiung, Kaohsiung (TW)
(74) Representative: Franks, Barry Gerard

(56) References cited:
- WO-A1-2008/086004
- WO-A2-2005/123160
- US-A- 3 712 302
- US-A- 4 659 330
- US-A- 5 514 099

## Description

### 1. Field of the Invention

The present invention relates to a needle cap, and more particularly to a safety needle cap that can be operated and recovered safely and can prevent a used needles from being reused.

### 2. Description of Related Art

Needles can be classified, according to usage, into syringe needles, intravenous needles, scalp needles or blood drawing needles. A needle cap is mounted around a tip of the needle to prevent accidental contamination of the needle during transportation, handling or after use. In use, the needle is held by one hand and the needle cap is removed by the other hand. Although the conventional needle cap can provide a covering effect for the needle, the tip of the needle may injure the hand holding the conventional needle cap, especially if the hand is accidentally jogged or knocked on removal or during replacement. Furthermore, a used needles may be reused accidentally so risking disease transmission. In addition, another conventional needle cap is disclosed in US 5.514,099 which discloses the features of the preamble of claim 1.

In order to improve the safety of using the needle cap, a safety needle cap is proposed that can be removed and replaced safely while also preventing reuse of a needle to overcome the aforementioned shortcomings.

### IN THE DRAWINGS:

Fig. 1 is a perspective view of a first embodiment of a safety needle cap in accordance with the present invention mounted around a syringe needle;
Fig. 2 is an exploded perspective view of a second embodiment of a safety needle cap in accordance with the present invention with an intravenous needle;

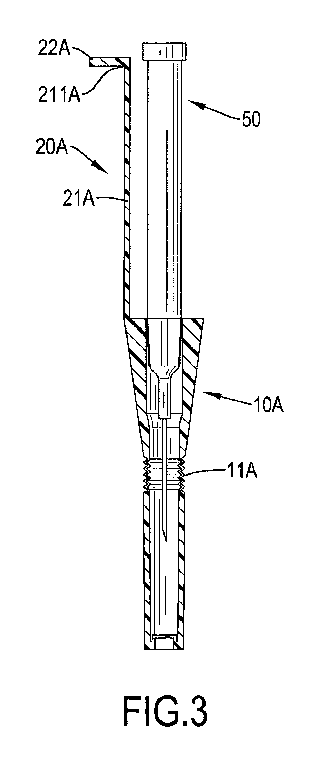

Fig. 3 is an enlarged side view in partial section of the safety needle cap in Fig. 2 mounted around the intravenous needle;
Fig. 4 is an operational side view in partial section of the safety needle cap in Fig. 3;
Fig. 5 is a perspective view of a third embodiment of a safety needle cap in accordance with the present invention;
Fig. 6 is an exploded perspective view of a fourth embodiment of a safety needle cap in accordance with the present invention with a scalp needle;
Fig. 7 is a perspective view of a fifth embodiment of a safety needle cap in accordance with the present invention mounted around a syringe needle;
Fig. 8 is an operational side view of the safety needle cap in Fig. 7;
Fig. 9 is a side view in partial section of a sixth embodiment of a safety needle cap in accordance with the present invention mounted around a large size syringe needle; and
Fig. 10 is a side view in partial section of a seventh embodiment of a safety needle cap in accordance with the present invention mounted around a large size syringe needle.

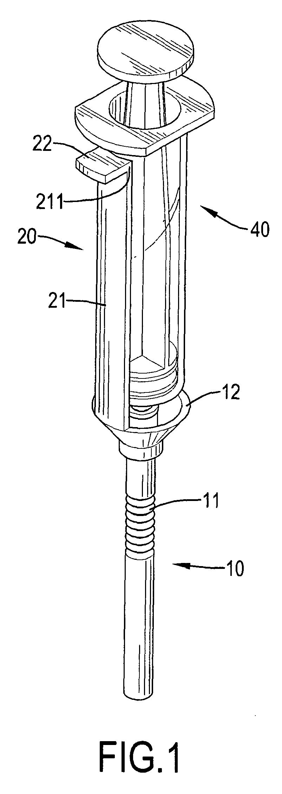

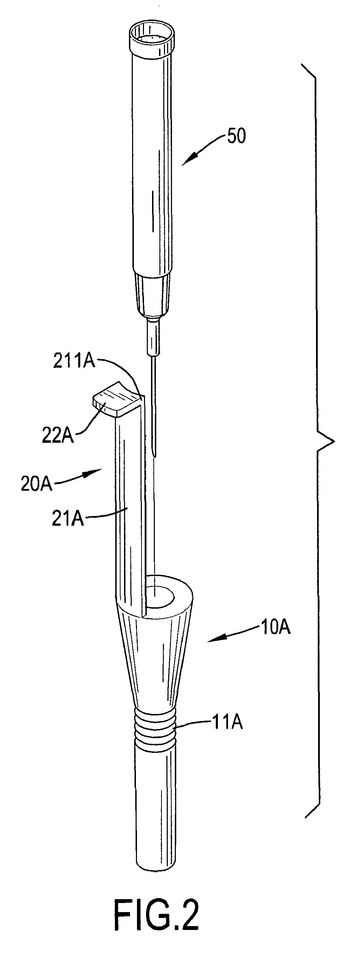

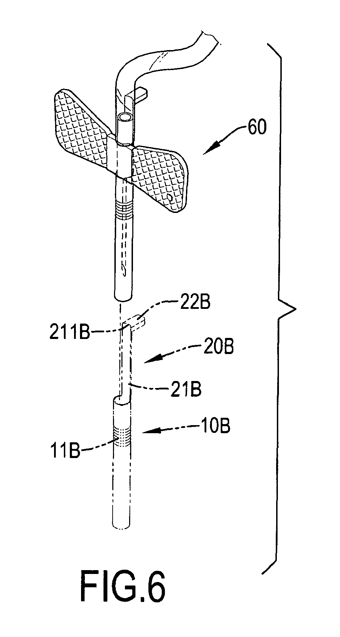

With reference to Figs. 1, 2, 5 to 7, 9 and 10, a safety needle cap in accordance with the present invention as defined in claim 1 comprises a body (10, 10A, 10B, 10C, 10D, 10E) and at least one holding segment (20, 20A, 20B, 20C, 20D, 20E).

The body (10, 10A, 10B, 10C, 10D, 10E) is tubular, is mounted around a syringe needle (40), an intravenous needle (50), a scalp needle (60) or a large size syringe needle (70) as shown in Figs. 1, 2, 6, 7, 9 and 10, has an external surface, a closed end, an open end, a chamber, a diameter and a bending segment (11, 11A, 11B, 11C) and may have a top cover (12, 12C). The chamber is defined in the body (10, 10A, 10B, 10C, 10D, 10E) between the closed end and the open end to contain the needle (40, 50, 60, 70).

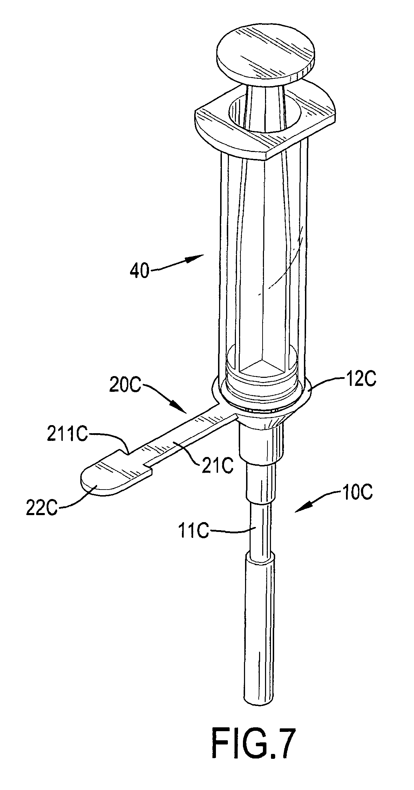

As defined in the characterising part of claim 1 the bending segment (11, 11A, 11B, 11C) is formed on the external surface of the body (10, 10A, 10B, 10C) to make the body (10, 10A, 10B, 10C) bendable. With reference to Figs. 1, 2, 5 and 6, the bending segment (11, 11A, 11B) is formed on the external surface of the body (10, 10A, 10B) near the open end and is a shrinking structure. Preferably, with reference to Fig. 7, the bending segment (11C) is annularly formed on the external surface of the body (10C) and has a diameter smaller than the diameter of the body (10C) to form a step bending structure between the closed end and the open end of the body (10C) to face an outer surface of the needle (40). With reference to Figs. 1, 7 and 9, the top cover (12, 12C) is funnel shaped and is formed on and protrudes from the open end of the body (10, 10C).

The at least one holding segment (20, 20A, 20B, 20C, 20D, 20E) is formed on the body (10, 10A, 10B, 10C, 10D, 10E) and each one of the at least one holding segment (20, 20A, 20B, 20C, 20D, 20E) has an extending stem (21, 21A, 21B, 21C, 21D, 21E) and a pulling tab (22, 22A, 22B, 22C, 22D, 22E).

With reference to Figs. 1, 2, 5 to 7 and 9, the extending stem (21, 21A, 21B, 21C, 21D) is formed on the open end of the body (10, 10A, 10B, 10C, 10D) and has a free end (211, 211A, 211B, 211C, 211D) opposite to the open end of the body (10, 10A, 10B, 10C, 10D). The extending stem (21, 21A, 21B, 21D) is longitudinally formed on and protrudes from the open end of the body (10, 10A, 10B, 10D) as shown in Figs, 1, 2, 6 and 9 or the extending stem (21C) is transversally formed on and protrudes from the open end of the body (10C) as shown in Fig. 7. With reference to Figs. 1 and 7, the extending stem (21) is longitudinally formed on the top cover (12) of the body (10) or the extending stem (21C) and is transversally formed on the top cover (12C) of the body (10C).

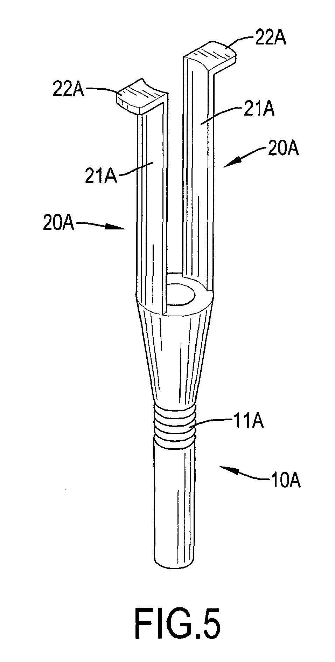

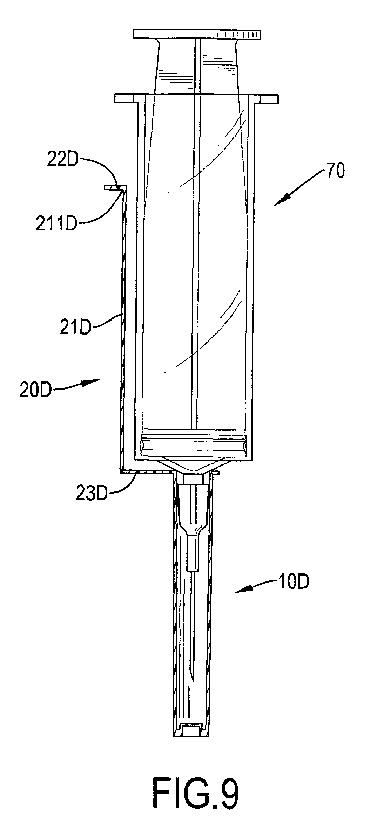

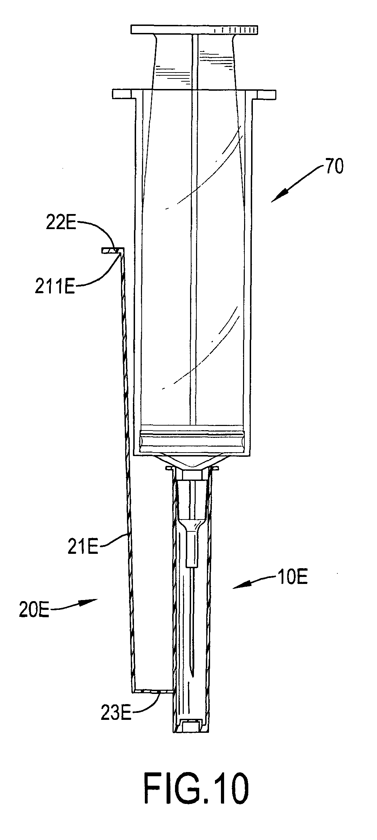

Preferably, with reference to Fig. 5, the safety needle cap has two holding segments (20A) longitudinally formed on and protruding from the open end of the body (10A). With reference to Fig. 10, the extending stem (21E) is formed on the external surface of the body (10E) between the open end and the closed end and has a free end (211E). Preferably, with further reference to Figs. 9 and 10, the extending stem (21D, 21E) has a connecting panel (23D, 23E) transversally formed on the extending stem (21D, 21E) at an end opposite to the free end (211D, 211E) and connected to the body (10D, 10E) to prevent the extending stem (21D, 21E) from interfering with the large size syringe needle (70).

The pulling tab (22, 22A, 22B, 22C, 22D, 22E) is formed on the free end (211, 211A, 211B, 211C, 211D, 211E) of the extending stem (21, 21A, 21B, 21C, 21D, 21E) to allow the extending stem (21, 21A, 21B, 21C, 21D, 21E) to be pulled easily.

In use, with reference to Figs. 1, 2, 6, 7, 9 and 10, the needle (40, 50, 60, 70) is removed from the safety needle cap in accordance with the present invention, one hand holds the pulling tab (22, 22A, 22B, 22C, 22D, 22E) of the holding segment (20, 20A, 20B, 20C, 20D, 20E) and the other hand pulls the needle (40, 50, 60, 70) out of the body (10, 10A, 10B, 10C, 10D, 10E). During removal, direct contact between the hand that holds the pulling tab (22, 22A, 22B, 22C, 22D, 22E) and the needle (40, 50, 60, 70) is prevented by the extending stem (21, 21A, 21B, 21C, 21D, 21E). In other words, the hand that holds the pulling tab (22, 22A, 22B, 22C, 22D, 22E) is kept away from the tip of the needle (40, 50, 60, 70). Then, the hand that holds the pulling tab (22, 22A, 22B, 22C, 22D, 22E) or the free end of the extending stem (21, 21A, 21B, 21C, 21D, 21E) is prevented from injury by the extending stem (21, 21A, 21B, 21C, 21D, 21E) even if either hand is accidentally knocked or jogged when removing or replacing the needle (40, 50, 60, 70).

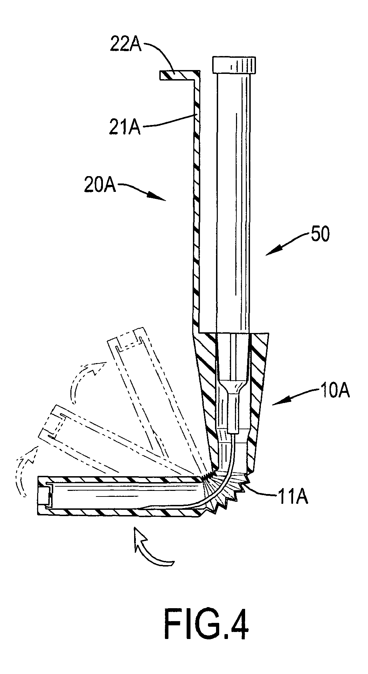

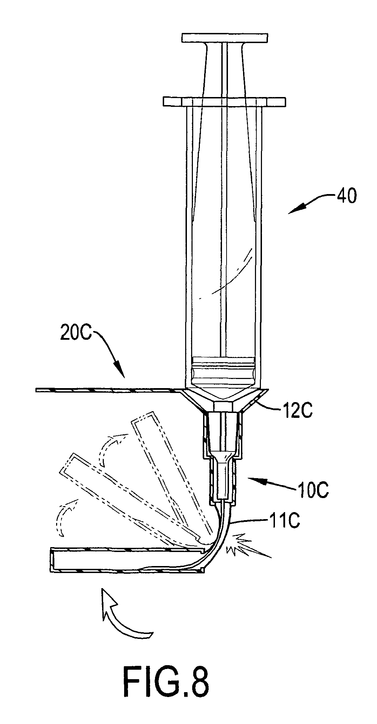

After use, the needle (40, 50, 60, 70) is replaced in the needle cap and the tip of the needle (40) is guided into the chamber of the body (10, 10A, 10B, 10C, 10D, 10E) by the extending stem (21, 21A, 21B, 21C, 21D, 21E) of the holding segment (20, 20A, 20B, 20C, 20D, 20E). Accordingly, the hand that holds the pulling tab (22, 22A, 22B, 22C, 22D, 22E) or the free end of the extending stem (21, 21A, 21B, 21C, 21D, 21E) is kept from contacting the tip of the needle (40, 50, 60, 70). After the used needle (40, 50, 60) is mounted in the chamber of the body (10, 10A, 10B, 10C), the body (10, 10A, 10B, 10C) can be bent as shown in Figs. 4 and 8 by the bending segment (11, 11A, 11B, 11C) to bend the needle (40, 50, 60) in the chamber of the body (10, 10A, 10B, 10C). Thus, the used, bent needle (40, 50, 60) is prevented from being reused.

The safety needle cap can prevent the hand that holds the pulling tab (22, 22A, 22B, 22C, 22D, 22E) or the free end of the extending stem (21, 21A, 21B, 21C, 21D, 21E) from being injured by the tip of the needle (40, 50, 60, 70) and the bending segment (11, 11A, 11B, 11C) of the body (10, 10A, 10B, 10C) can provide a bending effect to the needle (40, 50, 60) to prevent the used needles (40, 50, 60) from being reused.

## Claims

1. A safety needle cap has a body (10, 10A, 10B, 10C, 10D, 10E) being tubular and having an external surface, a closed end, an open end, a chamber defined in the body (10, 10A, 10B, 10C, 10D, 10E) and a diameter; and at least one holding segment (20, 20A, 20B, 20C, 20D, 20E) formed on the body (10, 10A, 10B, 10C, 10D, 10E) and each one of the at least one holding segment (20, 20A, 20B, 20C, 20D, 20E) havingan extending stem (21, 21A, 21B, 21C, 21D, 21E) formed on the body (10, 10A, 10B, 10C, 10D, 10E) and having a free end (211, 211A, 211B, 211C, 211D, 211E) opposite to the body (10, 10A, 10B, 10C, 10D, 10E), **characterized in that** the safety needle cap comprises
the body (10, 10, 10B) having a bending segment (11, 11A, 11B) being a shrinking structure and formed on the external surface of the body (10, 10A, 10B) to make the body (10, 10A, 10 bendable.

2. The safety needle cap as claimed in claim 1, wherein each one of the at least one holding segment (20, 20A, 20B, 20C, 20D, 20E) has a pulling tab (22, 22A, 22B, 22C, 22D, 22E) formed on the free end (211, 211A, 211B, 211C, 211D, 211E) of the extending stem (21, 21A, 21B, 21C, 21D, 21E) to provide a pulling effect to the body (10, 10A, 10B, 10C, 10D, 10E).

3. The safety needle cap as claimed in claim 2, wherein the extending stem (21, 21A, 21B, 21D) of each one of the at least one holding segment (20, 20A, 20B, 20D) is longitudinally formed on and protrudes from the open end of the body (10, 10A, 10B, 10D).

4. The safety needle cap as claimed in claim 2, wherein the extending stem (21C) of each one of the at least one holding segment (20C) is transversally formed on and protrudes from the open end of the body (10C).

5. The safety needle cap as claimed in claim 2, wherein the extending stem (21E) of each one of the at least one holding segment (20E) is formed on the external surface of the body (10E) between the open end and the closed end.

6. The safety needle cap as claimed in claim 2, wherein
the body (10, 10C) has a funnel shaped top cover (12, 12C) formed on and protruding from the open end of the body (10, 10C); and
the extending stem (21) of each one of the at least one holding segment (20) is longitudinally formed on the top cover (12) of the body (10).

7. The safety needle cap as claimed in claim 2, wherein
the body (10C) has a funnel shaped top cover (12C) formed on and protruding from the open end of the body (10C); and
the extending stem (21 C) of each one of the at least one holding segment (20C) is transversally formed on the top cover (12C) of the body (10C).

8. The safety needle cap as claimed in claim 3, wherein the safety needle cap has two holding segments (21A) longitudinally formed on and protruding from the open end of the body (10A).

9. A safety needle cap claimed in claim 1 wherein a bending segment (11C) is formed annularly on the external surface of the body (10C) and having has a diameter smaller than the diameter of the body (10C) to form a step bending structure between the closed end and the open end of the body (10C) to face an outer surface of a needle.

10. The safety needle cap as claimed in claim 9, wherein each one of the at least one holding segment (20, 20A, 20B, 20C, 20D, 20E) has a pulling tab (22, 22A, 22B, 22C, 22D, 22E) formed on the free end (211, 211A, 211B, 211C, 211D, 211E) of the extending stem (21, 21A, 21B, 21C, 21D, 21E) to provide a pulling effect to the body 10 10A, 10B, 10C, 10D, 10E).

11. The safety needle cap as claimed in claim 10, wherein the extending stem (21, 21A, 21B, 21D) of each one of the at least one holding segment (20, 20A, 20B, 20D) is longitudinally formed on and protrudes from the open end of the body (10, 10A, 10B 10D).

12. The safety needle cap as claimed in claim 10, wherein the extending stem (21C) of each one of the at least one holding segment (20C) is transversally formed on and protrudes from the open end of the body (10C).

13. The safety needle cap as claimed in claim 10, wherein the extending stem (21E) of each one of the at least one holding segment (20E) is formed on the external surface of the body (10E) between the open end and the closed end.

14. The safety needle cap as claimed in claim 10, wherein
the body (10, 10C) has a funnel shaped top cover (12, 12C) formed on and protruding from the open end of the body (10, 10C); and
the extending stem (21) of each one of the at least one holding segment (20) is longitudinally formed on the top cover (12) of the body (10).

15. The safety needle cap as claimed in claim 10 wherein
the body (10C) has a funnel shaped top cover (12C) formed on and protruding from the open end of the body (10C) and
the extending stem (21C) of each one of the at least one holding segment (20C) is transversally formed on the top cover (12C) of the body (10C).

16. The safety needle cap as claimed in claim 11, wherein the safety needle cap has two holding segments (21A) longitudinally formed on and protruding from the open end of the body (10A).

## Patentansprüche

1. Sicherheits-Nadelkappe mit einem Körper (10, 10A, 10B, 10C, 10D, 10E), der röhrenförmig ist und eine Außenfläche, ein geschlossenes Ende, ein offenes Ende, eine im Körper (10, 10A, 10B, 10C, 10D, 10E) gebildete Kammer und einen Durchmesser hat, und mindestens einem Haltesegment (20, 20A, 20B, 20C, 20D, 20E), das am Körper (10, 10A, 10B, 10C, 10D, 10E) gebildet ist, und wobei jedes des mindestens einen Haltesegments (20, 20A, 20B, 20C, 20D, 20E) einen Verlängerungsstiel (21, 21A, 21B, 21C, 21D, 21E) hat, der am Körper (10, 10A, 10B, 10C, 10D, 10E) gebildet ist, und ein freies Ende (211, 211A, 211B, 211C, 211D, 211E) entgegengesetzt zum Körper (10, 10A, 10B, 10C, 10D, 10E) hat, **dadurch gekennzeichnet, dass** die Sicherheits-Nadelkappe aufweist:
den Körper (10, 10A, 10B) mit einem Biegesegment (11, 11A, 11B), das ein Schrumpfaufbau ist und auf der Außenfläche des Körpers (10, 10A, 10B) gebildet ist, um den Körper (10, 10A, 10B) biegbar zu machen.

2. Sicherheits-Nadelkappe nach Anspruch 1, wobei jedes des mindestens einen Haltesegments (20, 20A, 20B, 20C, 20D, 20E) eine Zuglasche (22, 22A, 22B, 22C, 22D, 22E) hat, die am freien Ende (211, 211A, 211B, 211C, 211D, 211E) des Verlängerungsstiels (21, 21A, 21B, 21C, 21D, 21E) gebildet ist, um für eine Zugwirkung auf den Körper (10, 10A, 10B, 10C, 10D, 10E) zu sorgen.

3. Sicherheits-Nadelkappe nach Anspruch 2, wobei der Verlängerungsstiel (21, 21A, 21B, 21D) jedes des mindestens einen Haltesegments (20, 20A, 20B, 20D) auf dem offenen Ende des Körpers (10, 10A, 10B, 10D) längs gebildet ist und davon vorsteht.

4. Sicherheits-Nadelkappe nach Anspruch 2, wobei der Verlängerungsstiel (21C) jedes des mindestens einen Haltesegments (20C) auf dem offenen Ende des Körpers (10C) quer gebildet ist und davon vorsteht.

5. Sicherheits-Nadelkappe nach Anspruch 2, wobei der Verlängerungsstiel (21E) jedes des mindestens einen Haltesegments (20E) auf der Außenfläche des Körpers (10E) zwischen dem offenen Ende und dem geschlossenen Ende gebildet ist.

6. Sicherheits-Nadelkappe nach Anspruch 2, wobei
der Körper (10, 10C) eine trichterförmige obere Abdeckung (12, 12C) hat, die auf dem offenen Ende des Körpers (10, 10C) gebildet ist und davon vorsteht; und
der Verlängerungsstiel (21) jedes des mindestens einen Haltesegments (20) auf der oberen Abdeckung (12) des Körpers (10) längs gebildet ist.

7. Sicherheits-Nadelkappe nach Anspruch 2, wobei
der Körper (10C) eine trichterförmige obere Abdeckung (12C) hat, die auf dem offenen Ende des Körpers (10C) gebildet ist und davon vorsteht; und
der Verlängerungsstiel (21C) jedes des mindestens einen Haltesegments (20C) auf der oberen Abdeckung (12C) des Körpers (10C) quer gebildet ist.

8. Sicherheits-Nadelkappe nach Anspruch 3, wobei die Sicherheits-Nadelkappe zwei Haltesegmente (21A) hat, die auf dem offenen Ende des Körpers (10A) längs gebildet sind und davon vorstehen.

9. Sicherheits-Nadelkappe nach Anspruch 1, wobei ein Biegesegment (11C) auf der Außenfläche des Körpers (10C) ringförmig gebildet ist und einen kleineren Durchmesser als der Durchmesser des Körpers (10C) hat, um einen Stufenbiegeaufbau zwischen dem geschlossenen Ende und dem offenen Ende des Körpers (10C) so zu bilden, dass er zu einer Außenfläche einer Kanüle weist.

10. Sicherheits-Nadelkappe nach Anspruch 9, wobei jedes des mindestens einen Haltesegments (20, 20A, 20B, 20C, 20D, 20E) eine Zuglasche (22, 22A, 22B, 22C, 22D, 22E) hat, die am freien Ende (211, 211A, 211B, 211C, 211D, 211E) des Verlängerungsstiels (21, 21A, 21B, 21C, 21D, 21E) gebildet ist, um für eine Zugwirkung auf den Körper (10, 10A, 10B, 10C, 10D, 10E) zu sorgen.

11. Sicherheits-Nadelkappe nach Anspruch 10, wobei der Verlängerungsstiel (21, 21A, 21B, 21D) jedes des mindestens einen Haltesegments (20, 20A, 20B, 20D) auf dem offenen Ende des Körpers (10, 10A, 10B, 10D) längs gebildet ist und davon vorsteht.

12. Sicherheits-Nadelkappe nach Anspruch 10, wobei der Verlängerungsstiel (21C) jedes des mindestens einen Haltesegments (20C) auf dem offenen Ende des Körpers (10C) quer gebildet ist und davon vorsteht.

13. Sicherheits-Nadelkappe nach Anspruch 10, wobei der Verlängerungsstiel (21E) jedes des mindestens einen Haltesegments (20E) auf der Außenfläche des Körpers (10E) zwischen dem offenen Ende und dem geschlossenen Ende gebildet ist.

14. Sicherheits-Nadelkappe nach Anspruch 10, wobei
der Körper (10, 10C) eine trichterförmige obere Abdeckung (12, 12C) hat, die auf dem offenen Ende des Körpers (10, 10C) gebildet ist und davon vorsteht; und
der Verlängerungsstiel (21) jedes des mindestens einen Haltesegments (20) auf der oberen Abdeckung (12) des Körpers (10) längs gebildet ist.

15. Sicherheits-Nadelkappe nach Anspruch 10, wobei
der Körper (10C) eine trichterförmige obere Abdeckung (12C) hat, die auf dem offenen Ende des Körpers (10C) gebildet ist und davon vorsteht; und
der Verlängerungsstiel (21C) jedes des mindestens einen Haltesegments (20C) auf der oberen Abdeckung (12C) des Körpers (10C) quer gebildet ist.

16. Sicherheits-Nadelkappe nach Anspruch 11, wobei die Sicherheits-Nadelkappe zwei Haltesegmente (21A) hat, die auf dem offenen Ende des Körpers (10A) längs gebildet sind und davon vorstehen.

## Revendications

1. Capuchon d'aiguille de sécurité, comprenant un corps (10, 10A, 10B, 10C, 10D, 10E) de forme tubulaire et présentant une surface externe, une extrémité fermée, une extrémité ouverte, une chambre, définie dans le corps (10, 10A, 10B, 10C, 10D, 10E) et un diamètre et au moins un segment de maintien (20, 20A, 20B, 20C, 20D, 20E) formé sur le corps (10, 10A, 10B, 10C, 10D, 10E), et chacun des au moins un segment de maintien (20, 20A, 20B, 20C, 20D, 20E) comprenant une tige d'extension (21, 21A, 21B, 21C, 21D, 21E) formé sur le corps (10, 10A, 10B, 10C, 10D, 10E) et ayant une extrémité libre (211, 211A, 211B, 211C, 211D, 211E) opposée au corps (10, 10A, 10B, 10C, 10D, 10E), **caractérisé en ce que** le capuchon d'aiguille de sécurité est tel que :
le corps (10, 10A, 10B) présente un segment de cintrage (11, 11A, 11B), réalisé sous forme de structure à rétrécissement et formé sur la surface externe du corps (10, 10A, 10B), pour rendre le corps (10, 10A, 10B) susceptible de se couder.

2. Capuchon d'aiguille de sécurité selon la revendication 1, dans lequel chacun des au moins un segment de maintien (20, 20A, 20B, 20C, 20D, 20E) comprend une patte de traction (22, 22A, 22B, 22C, 22D, 22E) formée sur l'extrémité libre (211, 211A, 211B, 211C, 211 D, 211 E) de la tige d'extension (21, 21A, 21B, 21C, 21D, 21E), pour fournir un effet de traction au corps (10, 10A, 10B, 10C, 10D, 10E).

3. Capuchon d'aiguille de sécurité selon la revendication 2, dans lequel la tige d'extension (21, 21A, 21B, 21D) de chacun des au moins un segment de maintien (20, 20A, 20B, 20D) est formée longitudinalement sur et fait saillie de l'extrémité ouverte du corps (10, 10A, 10B, 10D).

4. Capuchon d'aiguille de sécurité selon la revendication 2, dans lequel la tige d'extension (21C) de chacun des au moins un segment de maintien (20C) est formée transversalement sur et fait saillie de l'extrémité ouverte du corps (10C).

5. Capuchon d'aiguille de sécurité selon la revendication 2, dans lequel la tige d'extension (21 E) de chacun des au moins un segment de maintien (20E) est formée sur la surface externe du corps (10E), entre l'extrémité ouverte et l'extrémité fermée.

6. Capuchon d'aiguille de sécurité selon la revendication 2, dans lequel
le corps (10, 10C) présente un couvercle supérieur (12, 12C) en forme d'entonnoir, formé sur et faisant saillie de l'extrémité ouverte du corps (10, 10C) ; et
la tige d'extension (21) de chacun des au moins un segment de maintien (20) est formée longitudinalement sur le couvercle supérieur (12) du corps (10).

7. Capuchon d'aiguille de sécurité selon la revendication 2, dans lequel
le corps (10C) présente un couvercle supérieur (12C) en forme d'entonnoir, formé sur et faisant saillie de l'extrémité ouverte du corps (10C) ; et
la tige d'extension (21 C) de chacun des au moins un segment de maintien (20C) est formée transversalement sur le couvercle supérieur (12C) du corps (10C).

8. Capuchon d'aiguille de sécurité selon la revendication 3, dans lequel le capuchon d'aiguille de sécurité comprend deux segments de maintien (21A), formés longitudinalement sur et faisant saillie de l'extrémité ouverte du corps (10A).

9. Capuchon d'aiguille de sécurité selon la revendication 1, dans lequel un segment de cintrage (11C) est formé de manière annulaire sur la surface externe du corps (10C) et présente un diamètre plus petit que le diamètre du corps (10C), de manière à former une structure de cintrage à rétreint, entre l'extrémité fermée et l'extrémité ouverte du corps (10C), et à faire face à une surface extérieure d'une aiguille,

10. Capuchon d'aiguille de sécurité selon la revendication 9, dans lequel chacun des au moins un segment de maintien (20, 20A, 20B, 20C, 20D, 20E) comprend une patte de traction (22, 22A, 22B, 22C, 22D, 22E) formée sur l'extrémité libre (211, 211A, 211B, 211C, 211D, 211E) de la tige d'extension (21, 21A, 21B, 21C, 21D, 21E), pour fournir un effet de traction au corps (10, 10A, 10B, 10C, 10D, 10E).

11. Capuchon d'aiguille de sécurité selon la revendication 10, dans lequel la tige d'extension (21, 21A, 21B, 21D) de chacun des au moins un segment de maintien (20, 20A, 20B, 20D) est formée longitudinalement sur et fait saillie de l'extrémité ouverte du corps (10, 10A, 10B, 10D).

12. Capuchon d'aiguille de sécurité selon la revendication 10, dans lequel la tige d'extension (21C) de chacun des au moins un segment de maintien (20C) est formée transversalement sur et fait saillie de l'extrémité ouverte du corps (10C).

13. Capuchon d'aiguille de sécurité selon la revendication 10, dans lequel la tige d'extension (21E) de chacun des au moins un segment de maintien (20E) est formée sur la surface externe du corps (10E), entre l'extrémité ouverte et l'extrémité fermée.

14. Capuchon d'aiguille de sécurité selon la revendication 10, dans lequel
le corps (10, 10C) présente un couvercle supérieur (12, 12C) en forme d'entonnoir, formé sur et faisant saillie de l'extrémité ouverte du corps (10, 10C) ; et
la tige d'extension (21) de chacun des au moins un segment de maintien (20) est formée longitudinalement sur le couvercle supérieur (12) du corps (10).

15. Capuchon d'aiguille de sécurité selon la revendication 10, dans lequel
le corps (10C) présente un couvercle supérieur (12C) en forme d'entonnoir, formé sur et faisant saillie de l'extrémité ouverte du corps (10C) ; et
la tige d'extension (21C) de chacun des au moins un segment de maintien (20C) est formée transversalement sur le couvercle supérieur (12C) du corps (10C).

16. Capuchon d'aiguille de sécurité selon la revendication 11, dans lequel le capuchon d'aiguille de sécurité comprend deux segments de maintien (21A), formés longitudinalement sur et faisant saillie de l'extrémité ouverte du corps (10A).
